(19) Europäisches Patentamt

European Patent Office

Office européen des brevets

(11) Veröffentlichungsnummer: **0 298 190 B1**

(12) **EUROPÄISCHE PATENTSCHRIFT**

(45) Veröffentlichungstag der Patentschrift: **27.05.92**

(21) Anmeldenummer: **88102661.1**

(22) Anmeldetag: **24.02.88**

(51) Int. Cl.⁵: **A61K 6/04**, A61C 13/08

Die Akte enthält technische Angaben, die nach dem Eingang der Anmeldung eingereicht wurden und die nicht in dieser Patentschrift enthalten sind.

(54) Dental-Verbundwerkstoff und Verfahren zur Herstellung eines solchen Verbundwerkstoffes.

(30) Priorität: **09.06.87 DD 303602**

(43) Veröffentlichungstag der Anmeldung:
**11.01.89 Patentblatt 89/02**

(45) Bekanntmachung des Hinweises auf die
Patenterteilung:
**27.05.92 Patentblatt 92/22**

(84) Benannte Vertragsstaaten:
**AT CH DE FR IT LI**

(56) Entgegenhaltungen:
**EP-A- 0 151 233**
**DE-A- 3 212 345**

**CHEMICAL ABSTRACTS, Band 107, Nr. 16, 19 Oktober 1987, Columbus, OH (US); H.HERO et al., Seite 438, Nr. 141069u**

**PATENT ABSTRACTS OF JAPAN, Band 8, Nr. 117 (P-277)[1554], 31 Mai 1984**

(73) Patentinhaber: **Heraeus Kulzer GmbH**
**Heraeusstr. 12 - 14**
**W-6450 Hanau(DE)**

(72) Erfinder: **Schmidt, Albert**
**Heuchelheimer Str. 139a**
**W-6380 Bad Homburg(DE)**
Erfinder: **Tiller, Hans-Jürgen, Dr.**
**Prof. Ibrahim-Str. 23**
**DD-6900 Jena(DE)**
Erfinder: **Göbel, Roland, Dr.**
**Heimstättenstr. 12**
**DD-6900 Jena(DE)**
Erfinder: **Wowra, Hans-Jürgen**
**Hohe Str. 26**
**DD-6570 Zeulenroda(DE)**
Erfinder: **Hilpmann, Bernd**
**Geraer Str. 20**
**DD-6576 Triebes(DE)**
Erfinder: **Magnus, Brigitte**
**Philipp Müllerstr. 4**
**DD-6900 Jena(DE)**

(74) Vertreter: **Grimm, Ekkehard**
**Heraeus Holding GmbH Heraeusstrasse 12 - 14**
**W-6450 Hanau/Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Dental-Verbundwerkstoff, insbesondere für Zahnersatz, mit einer Edelmetall enthaltenden Trägerschicht, deren Oberfläche mit einer silanisierten Silizium oder Siliziumoxid enthaltenden Deckschicht, die ihrerseits eine Kunststoff-Schicht trägt, beschichtet ist, sowie ein Verfahren zur Herstellung eines solchen Verbundwerkstoffes, bei dem auf eine Edelmetall enthaltende Trägerschicht zunächst eine silanisierte Silizium oder Siliziumoxid enthaltende Deckschicht und anschließend auf die Deckschicht eine Kunststoff-Schicht aufgebracht wird.

Eine Vorrichtung, die verfahrensgemäß betrieben wird und mit der solche Dental-Verbundwerkstoffe aufbaubar sind, ist aus der DE-OS 34 04 894 bekannt. Mittels einer solchen Vorrichtung werden edelmetallhaltige Dentalprothesenteile mit einer Silizium oder Siliziumoxid enthaltenden Schicht abgedeckt, bevor auf diese Deckschicht beispielsweise eine Verblendung aus Kunststoff aufgebracht wird. Durch diese Deckschicht wird eine große Haftfestigkeit des darauf aufgebrachten Kunststoffes erreicht.

Wie auch in der Beschreibungseinleitung der DE-PS 34 03 894 zum Stand der Technik angegeben ist, ist aus der US-PS 4 364 731 bekannt, metallische Dentalprothesenteile mit einer Siliziumoxid-Haftvermittlerschicht zu beschichten. Zur Beschichtung wird eine im Höchstfrequenzbereich arbeitende Magnetron-Sputtervorrichtung empfohlen, mit der das Siliziumoxid von hochreinem Quarzglas abgesputtert und in einer Vakuumkammer auf dem Dentalprothesenteil abgeschieden wird.

Die in der DE-PS 34 03 894 beschriebene Vorrichtung hat sich im praktischen Einsatz gut bewährt, da entsprechend beschichtete Dentalprothesenteile eine hohe Haftfestigkeit der Kunststoffschicht, beispielsweise einer Verblendschicht, die aus Opaker (Decklack) und Kunststoff besteht, zeigen.

Es hat sich herausgestellt, daß Dentalprothesenteile mit einer derartigen Kunststoff-Verblendung, die auf einem Träger mit einem hohen Anteil an Silber und Palladium aufgebaut sind und eine Silizium oder Siliziumoxid enthaltende Deckschicht aufweisen, zwar gegenüber Trägern ohne eine solche Deckschicht eine verbesserte Haftfestigkeit der darauf aufgebrachten Kunststoffschicht zeigen, jedoch nicht in der Größenordnung, die mit aus anderen Metallen aufgebauten Trägern erzielbar ist. Eine Erklärung für diese geringere Haftfestigkeit ist darin zu sehen, daß durch die hohen Temperaturen, die beim Aufbringen der Silizium oder Siliziumoxid enthaltenden Deckschicht mit einem Flammhydrolysebrenner kurzzeitig auf die obere Schicht des Trägers einwirken, eine Entmischung des Silber-Palladium-Verbundes auftritt. Diese verminderten Hafteigenschaften wurden insbesondere an Dentallegierungen beobachtet, die einen hohen Anteil an Silber im Bereich von 40 bis 75 Gewichts-% und einen Anteil von Palladium von 20 bis 30 Gewichts-% aufweisen.

Der vorliegenden Erfindung liegt nun die Aufgabe zugrunde, einen Dental-Verbundwerkstoff anzugeben, der gegenüber den bekannten Dental-Verbundwerkstoffen mit einem Träger mit einem hohen Anteil an Silber und Palladium eine erhöhte Haftfestigkeit der darauf aufzubringenden Kunststoffschicht zeigt, sowie ein entsprechendes Verfahren zur Herstellung eines solchen Dental-Verbundwerkstoffes aufzuzeigen.

Diese Aufgabe wird hinsichtlich eines Dental-Verbundwerkstoffes dadurch gelöst, daß der Träger (1) mindestens 20 Gewichts-% Silber und mindestens 20 Gewichts-% Palladium enthält und die Summe des Gewichtes an Palladium und Silber mindestens 50 Gewichts-% beträgt und daß zwischen der Oberfläche des Trägers (1) und der Deckschicht (3) eine mindestens 10 bis 150 nm dicke Wärmeschutzschicht (2) aus einem Metall der Gruppe Zinn, Chrom, Kupfer, Silber, Nickel, Zink oder Gold angeordnet ist.

Hinsichtlich des Verfahrens wird die Aufgabe dadurch gelöst, daß ein Träger, der mindestens 20 Gewichts-% Silber und mindestens 20 Gewichts-% Palladium enthält, bereitgestellt wird, wobei die Summe des Gewichtes an Palladium und Silber mindestens 50 Gewichts-% beträgt, und daß vor Aufbringen der Deckschicht eine zwischen 10 und 150 nm dicke Wärmeschutzschicht aus einem Metall der Gruppe Zinn, Chrom, Kupfer, Silber, Nickel, Zink oder Gold auf den Träger aufgebracht wird.

Mit dem angegebenen Aufbau wird ein Verbundwerkstoff erhalten, dessen Scherfestigkeitswerte weit oberhalb derjenigen liegen, die mit einem entsprechenden Verbundwerkstoff, dessen Oberfläche ebenfalls mit einer Silizium oder Siliziumoxid enthaltenden Deckschicht überzogen ist, erreicht werden können. Außerdem ist der Verbund randspaltfrei, d.h. es sind keine Spaltbildungen in der Grenzfläche nachweisbar. Die Scherfestigkeitswerte liegen bei einem erfindungsgemäßen Aufbau des Verbundwerkstoffes um einen Faktor 1,5 bis 2 und mehr höher als bei vergleichbaren Trägern, die keine entsprechende Wärmeschutzschicht aufweisen. Die erfindungsgemäß eingefügte Wärmeschutzschicht verhindert bei den hohen Temperaturen, die gerade beim Aufbringen einer Silizium oder Siliziumoxid enthaltenden Schicht mit einem Flammhydrolysebrenner auftreten werden, eine Entmischung des Silber-Palladium-Verbundes im oberen Schichtbereich des Trägers. Um einer Entmischung der Silber und Palladium enthaltenden Oberfläche des Trägers entgegenzuwirken, ist eine 10 bis 150 nm dicke Wärmeschutzschicht aus einem Metall der Gruppe Zinn, Chrom, Kupfer, Silber, Nickel, Zink oder Gold auf der Oberfläche des Trägers angeordnet.

Damit die Deckschicht, insbesondere dann, wenn sie mit einem Flammhydrolysebrenner aufgebracht wird, eine gute Haftfestigkeit auf der Wärmeschutzschicht erhält, ist es von Vorteil, die Oberfläche der Trägerschicht aufzurauhen. Die Oberflächenrauhigkeit sollte im Bereich von 1 bis 8 $\mu$m, bevorzugt im Bereich von 3 bis 5 $\mu$m liegen. Bei einer darauf aufgebrachten Wärmeschutzschicht von 10 bis 150 nm, bevorzugt von 15 bis 75 nm bzw. 20 bis 55 nm, zeigt die Oberfläche der Wärmeschutzschicht, auf der die Deckschicht aufgebracht wird, noch eine ausreichende Oberflächenrauhigkeit.

Bevorzugt besteht die Wärmeschutzschicht aus Zinn, Chrom oder Silber, die einerseits sehr gute Scherfestigkeitswerte ergeben und andererseits gerade im Dentalbereich verträglich sind. In diese Gruppe ist zusätzlich Gold mit einzuschließen. Besonders hohe Scherfestigkeitswerte wurden mit einer Wärmeschutzschicht aus Chrom erzielt, deren Dicke bevorzugt im Bereich von 30 bis 55 nm liegen sollte.

Gut bewährt hat sich eine Wärmeschutzschicht, die elektrolytisch auf den Träger aufgebracht wird. Diese Schicht zeichnet sich durch ihre gleichmäßige Dicke insbesondere dann aus, wenn der Träger an seiner Oberfläche aufgerauht ist, so daß die Rauhigkeit trotz dieser Wärmeschutzschicht erhalten bleibt.

Aus DE-A-32 12 345 sind dentale Verbundwerkstoffe, die ebenfalls eine auf elektrolytischem Wege hergestellte äußerst dünne metallische Zwischenschicht aufweisen, bekannt. Die Zwischenschicht wird auf mit Keramik zu verblendende Gußobjekte aus Dentallegierungen aufgebracht, um eine für die Haftung zwischen Legierung und Keramik optimale Grenzzone zu schaffen. Das Gußobjekt besteht dabei aus einer Edelmetall-, Nickel- oder Kobalt-Legierung und die Zwischenschicht aus Gold, Gold-Legierungen, Rhodium oder Palladium. Eine Zwischenschicht aus Gold kann auch dazu dienen, um mit Kunststoff oder Keramik zu verblendendem Zahnersatz aus weißen Edelmetall-Legierungen einen ästhetisch schöneren Farbton zu verleihen.

Weitere Einzelheiten und Merkmale der Erfindung ergeben sich aus der nachfolgenden Beschreibung verschiedener Ausführungsbeispiele. Die Zeichnung, auf die nachfolgend Bezug genommen wird, zeigt

Figur 1     den Schichtaufbau eines Dental-Verbundwerkstoffes gemäß der Erfindung,

Figur 2     eine Elektrolyseanordnung zur Aufbringung der Wärmeschutzschicht und

Figur 3     ein Diagramm, in dem die Scherfestigkeit in Abhängigkeit der Dicke einer Wärmeschutzschicht aus Chrom aufgetragen ist.

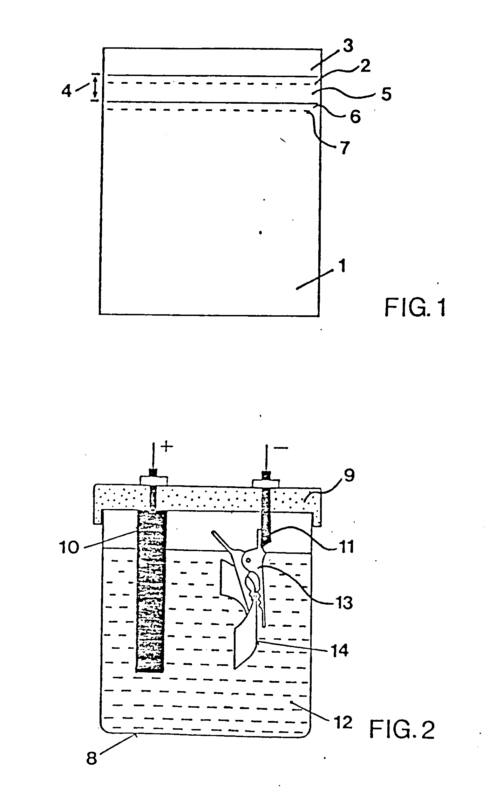

In Figur 1 ist der prinzipielle Aufbau eines Verbundwerkstoffes gezeigt mit einem Träger 1, der mindestens 20 Gewichts-% Palladium und mindestens 20 Gewichts-% Silber enthält und bei dem die Summe des Gewichtes an Palladium und Silber mindestens 50 Gewichts-% beträgt. Auf diesem Träger 1 ist eine Wärmeschutzschicht 2 angeordnet, auf der ihrerseits eine Deckschicht 3 aufgebracht ist. Die Wärmeschutzschicht 2, deren Dicke, durch den Pfeil 4 angedeutet, 10 bis 150 nm, bevorzugt 20 bis 55 nm, beträgt, besteht aus Zinn, Chrom, Kupfer, Silber, Nickel, Zink oder Gold, bevorzugt aus Zinn, Chrom oder Silber.

Im oberen Bereich der Wärmeschutzschicht 2 ist, durch die unterbrochene Linie 5 angedeutet, eine obere Schicht begrenzt, in der eine verstärkte Erwärmung beim Aufbringen der Deckschicht 3 durch einen Flammhydrolysebrenner auftritt. Der untere, dem Träger 1 zugewandte Bereich der Wärmeschutzschicht 2 wird nur auf eine Temperatur erwärmt, die unterhalb der Entmischungstemperatur des Trägers 1 aus Silber und Palladium, also unterhalb 600 °C, liegt. Ohne diese Wärmeschutzschicht 2 würde die Erwärmungszone in der oberen Schicht 6 des Trägers 1, durch die unterbrochene Linie 7 abgeteilt, liegen.

Die metallene Wärmeschutzschicht 2 muß eine solche Dicke aufweisen, daß eine thermische und chemische Abschirmung der Oberflächenschicht des Trägers 1 gewährleistet ist. Aus dieser Forderung ergibt sich eine Mindestdicke der Wärmeschutzschicht 2 von 10 nm. Durch die obere Grenze der Dicke der Wärmeschutzschicht 2 von 150 nm ist gewährleistet, daß die Eigenspannungen der Schicht in einem tolerierbaren Bereich liegen und die aufgerauhte Oberfläche des Trägers 1 erhalten bleibt.

Eine gleichmäßige Beschichtung der Oberfläche des Trägers 1 mit einer Wärmeschutzschicht ist auf elektrolytischem Wege möglich. Eine prinzipielle Elektrolyseanordnung zeigt Figur 2. Die Elektrolyseanordnung weist ein Elektrolysegefäß 8 (Durchmesser 10 cm, Höhe 8 cm), das mit einem Deckel 9 verschlossen ist, auf. Durch den Deckel 9 ist eine Kohle-Anode 10 (Durchmesser etwa 1,8 cm, Länge 6,5 cm) sowie eine Kathode 11 geführt. Eine solche Anode 10 aus Kohle hat sich als vorteilhaft erwiesen, da sie den Elektrolysevorgang durch ihr inertes Verhalten selbst nicht beeinflußt. Das Elektrolysegefäß 8 ist mit 300 ml Elektrolyt-Flüssigkeit 12 gefüllt. Am Ende der Kathode 11 ist eine Halteklammer 13 befestigt, in der zu galvanisierende Brücken oder Kronenteile 14 eingeklemmt werden können. Als Gleichstromversorgung wurde eine von 0 bis 12 V regelbare Spannungsquelle mit einer maximalen Stromstärke von 1 A eingesetzt. Mit dieser Anordnung wurden folgende Elektrolysebäder angewandt, und zwar auf einem Träger, der etwa 70 Gewichts-% Silber und etwa 30 Gewichts-% Palladium enthielt:

1. Chrom-Elektrolysebad

8,0 g pro 1 $H_2O$      $CrO_3$

2,0 g pro 1 $H_2O$      $K_2Cr_2O_7$

0,1 g pro 1 $H_2O$      Chromsulfat

Elektrolyseparameter:

Stromdichte:      10 mA cm$^{-2}$
Spannung:      10 V
Galvanisierzeit:      5 min

2. Zinn-Elektrolysebad

a) 15 g pro 1 Methanol $SnCl_2$
5 g pro 1 Methanol Phenol
b) 15 g pro 1 $H_2O$ $SnCl_2$
1 g pro 1 $H_2O$ HCl (37 %ig)
5 g pro 1 $H_2O$ Phenol
2 g pro 1 $H_2O$ Gelatine

Elektrolyseparameter:

Stromdichte:      1,7 mA cm$^{-2}$
Spannung:      10 V
Galvanisierzeit:      5 min

Die beschichteten Träger wurden anschließend mittels Flammhydrolysebrenner mit einer Siliziumoxidschicht überzogen, silanisiert, dann mit einer Opaker-Schicht ("Dentacolor® Opaker" der Firma Kulzer) abgedeckt und mit einer 3 mm dicken Kunststoffschicht (Dentacolor® (Dentacolor ist ein eingetragenes Warenzeichen der Firma Kulzer)) beschichtet. Diese Dental-Verbundwerkstoffe wurden auf ihre Randspaltfreiheit, nachdem sie einem Kochtest unterworfen wurden, untersucht. Im Rahmen dieses Kochhtestes wurden die Teile in einem mit Wasser gefüllten, offenen Gefäß 30 Minuten gekocht und auf Zimmertemperatur abgekühlt.

Folgende drei Versuche wurden durchgeführt:

A) Trägerschicht, auf die eine $SiO_x$-Schicht mit einem Flammhydrolysebrennner aufgebracht wurde, ohne Wärmeschutzschicht

B) Versuch wie A), jedoch mit einer elektrolytischen Verzinnung (Wärmeschutzschicht) auf der Trägerschicht

C) Versuch wie A), jedoch mit einer elektrolytischen Verchromung (Wärmeschutzschicht) auf der Trägerschicht

Erläuterung der aus den vorstehenden Versuchen gewonnenen Ergebnisse:

Zu Versuch A):      Es wurden partielle Spaltbildungen im Grenzbereich zwischen der Trägerschicht, der Opaker-Schicht und der Kunststoff-Schicht festgestellt.

Zu Versuch B):      Im Vergleich zu den Ergebnissen nach Versuch A) wurde eine verminderte Spaltbildung festgestellt.

Zu Versuch C):      Bei diesem Verbund konnte keine Spaltbildung nachgewiesen werden.

Die Verbundwerkstoffe nach Versuch B) und C) zeigten im Vergleich zu dem Verbundwerkstoff nach Versuch A) erhöhte Scherfestigkeitswerte.

In weiteren Versuchen wurden Wärmeschutzschichten aus unterschiedlichen Metallen auf gleichen Trägern, entsprechend der vorstehenden Zusammensetzung, miteinander verglichen. Die Dicke der Wärmeschutzschicht betrug 50 nm; die nachfolgende Probenpräparation erfolgte analog den vorstehenden Versuchen. Die Scherfestigkeitswerte wurden nach einem Kochtest, wie vorstehend aufgeführt, ermittelt.

| Material der Wärmeschutzschicht | Scherfestigkeit mit zusätzlicher $SiO_x$-C-Schicht (N/cm$^2$) |
|---|---|
| Zink | 1350 ± 10 % |
| Zinn | 1570 ± 10 % |
| Nickel | 1530 ± 10 % |
| Chrom | 1660 ± 10 % |

Verglichen mit den vorstehenden Werten von beschichteten Trägern, bei denen, wie aufgeführt, vier verschiedene Wärmeschutzschichten auf Trägerschichten untersucht werden, zeigten entsprechend aufgebaute Träger ohne eine Wärmeschutzschicht im Mittel eine Scherfestigkeit von 850 N/cm$^2$.

Wie die Zusammenstellung zeigt, konnten mit einer Wärmeschutzschicht aus Chrom die höchsten Scherfestigkeitswerte erreicht werden, gefolgt von einem Träger mit einer Wärmeschutzschicht aus Zinn und Nickel, wobei ein Träger mit Zink Scherfestigkeitswerte zeigte, die bis zu 20 % unter denjenigen lagen, die mit einem eine Chrom-Wärmeschutzschicht aufweisenden Träger erreicht werden konnten.

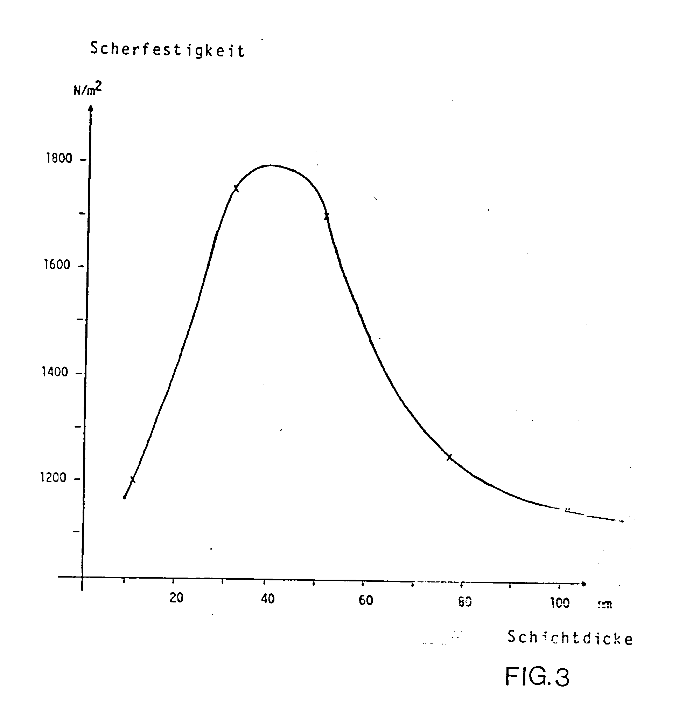

Weiterhin wurde die Abhängigkeit der Scherfestigkeit von der Schichtdicke für Chrom-Schichten überprüft. Die erhaltene Abhängigkeit ist in der Grafik in Figur 3 gezeigt.

Verglichen mit dem weiter oben angegeben Wert der Scherfestigkeit, der bei einem Träger mit einer 50 nm dicken Chrom-Schicht erzielt wurde, liegt das Maximum der erzielbaren Scherfestigkeit bei Schichtdikken zwischen 35 und 45 nm.

**Patentansprüche**

1. Dental-Verbundwerkstoff, insbesondere für Zahnersatz, mit einer Edelmetall enthaltenden Trägerschicht, deren Oberfläche mit einer silanisierten Silizium oder Siliziumoxid enthaltenden Deckschicht, die ihrerseits eine Kunststoff-Schicht trägt, beschichtet ist, dadurch gekennzeichnet, daß der Träger (1) mindestens 20 Gewichts-% Silber und mindestens 20 Gewichts-% Palladium enthält und die Summe des Gewichtes an Palladium und Silber mindestens 50 Gewichts-% beträgt und daß zwischen der Oberfläche des Trägers (1) und der Deckschicht (3) eine 10 bis 150 nm dicke Wärmeschutzschicht (2) aus einem Metall der Gruppe Zinn, Chrom, Kupfer, Silber, Nickel, Zink oder Gold angeordnet ist.

2. Dental-Verbundwerkstoff nach Anspruch 1, dadurch gekennzeichnet, daß die Dicke (4) der Wärmeschutzschicht (2) zwischen 15 und 75 nm beträgt.

3. Dental-Verbundwerkstoff nach Anspruch 1, dadurch gekennzeichnet, daß die Dicke (4) der Wärmeschutzschicht (2) zwischen 20 bis 55 nm beträgt.

4. Dental-Verbundwerkstoff nach einem der Ansprüche 1 bis 3, dadurch gekennzeichnet, daß die Wärmeschutzschicht (2) aus Zinn, Chrom oder Silber besteht.

5. Dental-Verbundwerkstoff nach Anspruch 4, dadurch gekennzeichnet, daß die Wärmeschutzschicht (2) eine Chrom-Schicht ist.

6. Dental-Verbundwerkstoff nach einem der Ansprüche 1 bis 5, dadurch gekennzeichnet, daß die Wärmeschutzschicht (2) eine elektrolytisch aufgebrachte Schicht ist.

7. Dental-Verbundwerkstoff nach einem der Ansprüche 1 bis 6, dadurch gekennzeichnet, daß die der Wärmeschutzschicht (2) zugewandte Oberfläche des Trägers (1) aufgerauht ist.

8. Dental-Verbundwerkstoff nach Anspruch 7, dadurch gekennzeichnet, daß die Oberflächenrauhigkeit im Bereich von 1 bis 8 $\mu$m liegt.

9. Dental-Verbundwerkstoff nach Anspruch 8, dadurch gekennzeichnet, daß die Oberflächenrauhigkeit im Bereich von 3 bis 5 $\mu$m liegt.

10. Dental-Verbundwerkstoff nach Anspruch 3 und 5, dadurch gekennzeichnet, daß die Wärmeschutzschicht (2) eine Chrom-Schicht ist mit einer Dicke (4) von 30 bis 55 nm.

11. Verfahren zur Herstellung eines Dental-Verbundwerkstoffes nach einem der Ansprüche 1 bis 11, bei dem auf einer Edelmetall enthaltenden Trägerschicht zunächst eine silanisierte Silizium oder Siliziumoxid enthaltende Deckschicht und anschließend auf die Deckschicht eine Kunststoff-Schicht aufgebracht wird, dadurch gekennzeichnet, daß ein Träger, der mindestens 20 Gewichts-% Silber und mindestens 20 Gewichts-% Palladium enthält, bereitgestellt wird, wobei die Summe des Gewichtes an Palladium und Silber mindestens 50 Gewichts-% beträgt und daß vor Aufbringen der Deckschicht eine zwischen 10 und 150 nm dicke Wärmeschutzschicht aus einem Metall der Gruppe Zinn, Chrom, Kupfer, Silber, Nickel, Zink oder Gold auf den Träger aufgebracht wird.

**12.** Verfahren nach Anspruch 11, dadurch gekennzeichnet, daß die Wärmeschutzschicht elektrolytisch aufgebracht wird.

**Claims**

**1.** A dental composite material, in particular for a tooth prosthesis with a carrier layer containing precious metal, the surface of which layer is coated with a covering layer containing silanised silicon or silicon dioxide, which covering layer, in turn, carries a layer of synthetic material, characterised in that the carrier (1) contains at least 20% -weight silver and at least 20% -weight palladium and the sum of the weight of palladium and silver is at least 50% -weight and that between the surface of the carrier (1) and of the covering layer (3) a heat insulation layer (2) is arranged which is 10 to 150 nm thick and is of a metal from the group tin, chromium, copper, silver, nickel, zinc or gold.

**2.** A dental composite material according to Claim 1, characterised in that the thickness (4) of the heat insulation layer (2) is between 15 and 75 nm.

**3.** A dental composite material according to Claim 1, characterised in that the thickness (4) of the heat insulation layer (2) is between 20 and 55 nm.

**4.** A dental composite material according to one of Claims 1 to 3, characterised in that the heat insulation layer (2) consists of tin, chromium or silver.

**5.** A dental composite material according to Claim 4, characterised in that the heat insulation layer (2) is a chromium layer.

**6.** A dental composite material according to one of Claims 1 to 5, characterised in that the heat insulation layer (2) is a layer which is deposited electrolytically.

**7.** A dental composite material according to one of Claims 1 to 6, characterised in that the surface of the carrier (1) facing the heat insulation layer (2) is roughened.

**8.** A dental composite material according to Claim 7, characterised in that the roughness of the surface lies in the range of 1 to 8 $\mu$m.

**9.** A dental composite material according to Claim 8, characterised in that the roughness of the surface lies in the range of 3 to 5 $\mu$m.

**10.** A dental composite material according to Claims 3 and 5, characterised in that the heat insulation layer (2) is a chromium layer with a thickness (4) of 30 to 55 nm.

**11.** A method for the production of a dental composite material according to one of Claims 1 to 10 in which firstly a covering layer containing silanised silicon or silicon dioxide is applied on a carrier layer containing precious metal, and then a layer of synthetic material is applied onto the covering layer, characterised in that a carrier, which contains at least 20% -weight silver and at least 20% -weight palladium, is prepared, in which the sum of the weight of palladium and silver is at least 50% -weight and that before the application of the covering layer, a heat insulation layer, between 10 and 150 nm thick, of a metal from the group tin, chromium, copper, silver, nickel, zinc or gold, is applied onto the carrier.

**12.** A method according to Claim 11, characterised in that the heat insulation layer is deposited electrolytically.

**Revendications**

**1.** Matériau composite dentaire, en particulier pour prothèses dentaires, comportant une couche support contenant un métal noble dont la surface est revêtue d'une couche de recouvrement contenant du silicium ou de l'oxyde de silicium silanisé et portant elle-même une couche de matière plastique, caractérisé en ce que le support (1) contient au moins 20 % en poids d'argent et au moins 20 % en poids de palladium et la somme du poids de palladium et du poids d'argent est d'au moins 50 % en poids et en ce qu'une couche de protection thermique (2) en un métal du groupe de l'étain, du chrome, du cuivre, de l'argent, du nickel, du zinc ou de l'or épaisse de 10 à 150 nm est disposée entre la surface du support (1) et la couche de recouvrement (3).

2.   Matériau composite dentaire selon la revendication 1, caractérisé en ce que l'épaisseur (4) de la couche de protection thermique (2) est de 15 à 75 nm.

3.   Matériau composite dentaire selon la revendication 1, caractérisé en ce que l'épaisseur (4) de la couche de protection thermique (2) est de 20 à 55 nm.

4.   Matériau composite dentaire selon l'une des revendications 1 à 3, caractérisé en ce que la couche de protection thermique (2) consiste en étain, en chrome ou en argent.

5.   Matériau composite dentaire selon la revendication 4, caractérisé en ce que la couche de protection thermique (2) est une couche de chrome.

6.   Matériau composite dentaire selon l'une des revendications 1 à 5, caractérisé en ce que la couche de protection thermique (2) est une couche appliquée par voie électrolytique.

7.   Matériau composite dentaire selon l'une des revendications 1 à 6, caractérisé en ce que la surface du support (1) qui est tournée vers la couche de protection thermique (2) est rendue rugueuse.

8.   Matériau composite dentaire selon la revendication 7, caractérisé en ce que la rugosité superficielle est située dans le domaine de 1 à 8 $\mu$m.

9.   Matériau composite dentaire selon la revendication 8, caractérisé en ce que la rugosité superficielle est située dans le domaine de 3 à 5 $\mu$m.

10.  Matériau composite dentaire selon les revendications 3 et 5, caractérisé en ce que la couche de protection thermique (2) est une couche de chrome d'une épaisseur (4) de 30 à 55 nm.

11.  Procédé de fabrication d'un matériau composite dentaire selon l'une des revendications 1 à 10, dans lequel une couche de recouvrement contenant du silicium ou de l'oxyde de silicium silanisé est tout d'abord appliquée sur une couche support contenant un métal noble puis une couche de matière plastique est appliquée sur la couche de recouvrement, caractérisé en ce que l'on prépare un support qui contient au moins 20 % en poids d'argent et au moins 20 % en poids de palladium, la somme du poids de palladium et du poids d'argent étant d'au moins 50 % en poids, et en ce qu'une couche de protection thermique épaisse de 10 à 150 nm en un métal du groupe de l'étain, du chrome, du cuivre, de l'argent, du nickel, du zinc ou de l'or est appliquée sur le support avant l'application de la couche de recouvrement.

12.  Procédé selon la revendication 11, caractérisé en ce que la couche de protection thermique est appliquée par voie électrolytique.

FIG. 1

FIG. 2

FIG.3